Europäisches Patentamt

European Patent Office

Office européen des brevets

(11) Publication number: 0 381 410 A1

## EUROPEAN PATENT APPLICATION

(21) Application number: 90300890.2

(22) Date of filing: 29.01.90

(51) Int. Cl.⁵: A61B 17/20, A61D 1/02

(30) Priority: 30.01.89 US 302928
06.07.89 US 375910

(43) Date of publication of application:
08.08.90 Bulletin 90/32

(84) Designated Contracting States:
AT BE CH DE DK ES FR GB GR IT LI LU NL SE

(71) Applicant: CONNAUGHT LABORATORIES LIMITED
1755 Steeles Avenue West
Willowdale Ontario M2R 3T4(CA)

(72) Inventor: Gilly, John A.
Rural Route 2, P.O. Box 289
Cresco, Pennsylvania 18326(US)
Inventor: Filipski, Ronald J.
R.D. 3, P.O. Box 3067A
East Stroudsburg, Pennsylvania 188301(US)

(74) Representative: Burford, Anthony Frederick et al
W.H. Beck, Greener & Co. 7 Stone Buildings
Lincoln's Inn
London WC2A 3SZ(GB)

(54) Clinical applicator.

(57) A clinical applicator or scarifier (10, 110) is provided with a marking means (24, 124) to mark the location of intradermal or subdermal injection of liquid antigen or vaccine, or other active substance, by tines (26, 126) at the time of injection.

FIG.3

EP 0 381 410 A1

## CLINICAL APPLICATOR

The present invention is concerned with a novel form of clinical applicator for application of liquid antigen or vaccine to the intradermal or subdermal region of skin.

The intradermal or subdermal application of liquid antigen or vaccine using scarifying devices having a plurality of tines or needles, usually to the forearm for testing or vaccination, particularly in children, is well known. The location of administration of the antigen or vaccine is important to know in cases where a reaction to the material administered demonstrates the presence of a disease. However, it is often difficult for the children and parents of the child to remember the location of administration.

A search conducted in the facilities of the United States Patent and Trademark Office has located the following U.S. Patents as the closest prior art:

U.S. 4,152,412 Brewer
U.S. 1,699,012 Naylor
U.S. 4,608,045 Fretwell
U.S. 3,167,073 Rosenthal
U.S. 3,351,059 Kravitz
U.S. 3,688,764 Reed

U.S. Patents Nos. 1,699,012 and 4,608,045 relate to animal markers in which an ink is applied to the skin of the animal for identification. Devices of various structures for transdermal injection are disclosed in U.S. Patents Nos. 3,167,073, 3,351,059, 3,688,764 and 4,453,926.

U.S. Patent No. 4,152,412 describes a vaccination system which provides visible evidence of such vaccination in cattle. In this prior art, vaccines, antigens, toxoids and the like are injected in conjunction with a physiologically acceptable colored particle of particle size such that the colored particle remains in the skin at the point of injection to provide a visible mark evidencing administration of the agent.

In accordance with the present invention, there is provided a clinical applicator comprising a plurality of needles for intradermal or subdermal administration of an active material, such as liquid antigen or vaccine, and visual image application means mounted on the applicator for applying a visual image adjacent the location of and simultaneously with the intradermal or subdermal administration.

By applying a visual image adjacent the location of administration, that location can more readily be identified at a future time. The visual image usually is applied as non-toxic ink, dye or other colored compound which resists washing off, so that the image is retained on the skin at the location of administration for some time. The application of the visual image adjacent to the site of administration does not interfere in any way with the application of the active material.

The visual image may be provided in the form of a design of any desired form. For example, for pediatric use, the design may be in the form of a "happy face" to provide a pleasing image to the child. Alternatively, the visual image may take the form of a simple ring for adult use.

The present invention contrasts markedly with the prior art by providing visible evidence of the site of administration of the agent without contaminating or otherwise interfering with the site of administration.

The antigens or vaccines which may be administered using the device of the present invention may be one of a variety of materials known to be administered using similar multi-tine devices, including tuberculin tests, tetanus sensitivity, diagnosis of histoplasmosis, blastomycosis, coccidimycosis, cryptococcisis, sporotrichosis, allergen sensitivity and smallpox vaccination.

The invention is described further, by way of illustration, with reference to the accompanying drawings, in which :

Figure 1 is a general perspective view of a clinical applicator or scarifying device constructed in accordance with one embodiment of the invention;

Figure 2 is a sectional view taken on line 2-2 of Figure 1;

Figure 3 is a perspective view of the device of Figure 1 with the cap member disassembled from the inoculator member;

Figure 4 is a sectional view taken on line 4-4 of Figure 3;

Figure 5 is the sectional view of the inoculator member of Figure 1 during application of active substance to skin; and

Figure 6 is a perspective view of applicator and skin showing the visual indication of the location of administration of the active substance;

Figure 7 is a general perspective view of a scarifying device, with the cap member removed from the inoculator member, in accordance with another embodiment of the invention;

Figure 8 is a sectional view of the clinical applicator of Figure 7;

Figure 9 is a perspective view of the clinical applicator of Figure 7, with the cap member assembled with the inoculator member; and

Figure 10 is a sectional view of the clinical applicator of Figure 7.

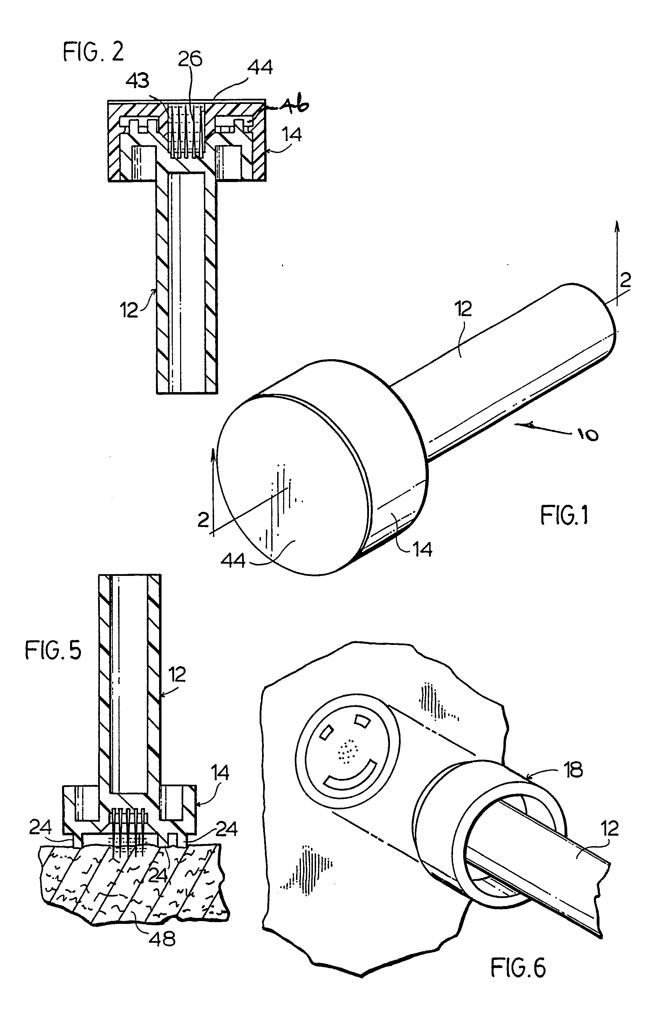

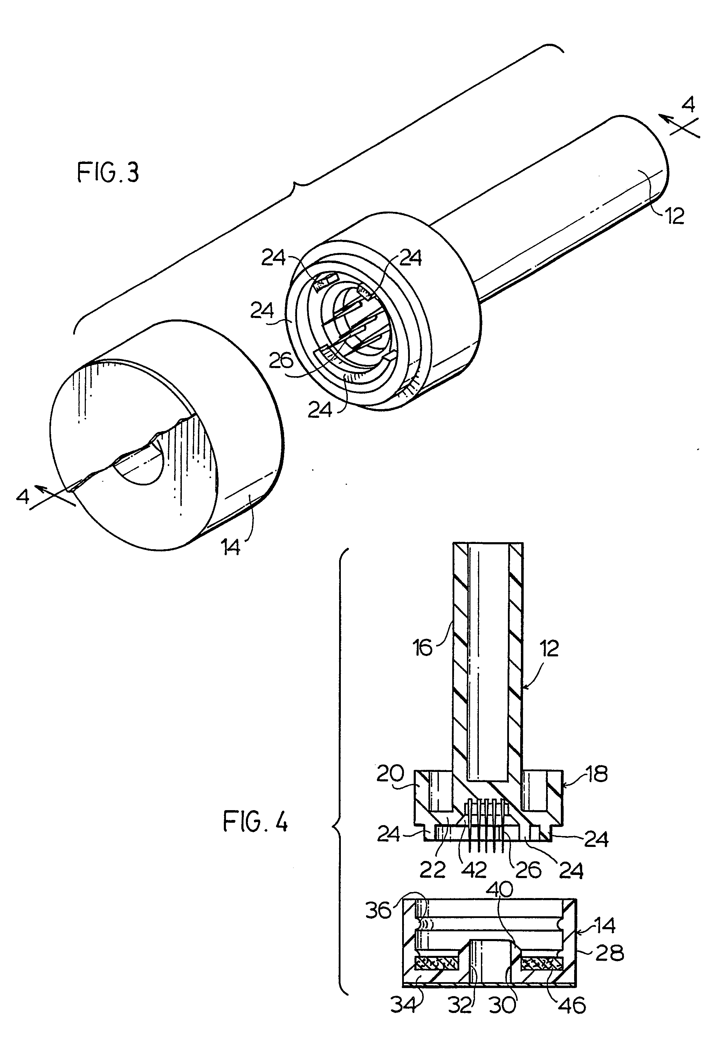

Referring first to the Figures 1 to 6 of the drawings, a scarifying device or clinical applicator 10 comprises an inoculator member 12 and a cap member 14. The inoculator member 12, which is a one-piece molded part, comprises an elongate handle 16 in the form of a hollow tube and a head 18 having a cylindrical outer flange or wall 20 joined to the handle 16 by a radially-directed flange or wall 22.

A series of projections 24 extend from the wall 22 in the shape and form of an identifying location indicating marking. A plurality of parallel needles or tines 26 protrude from the handle 16 beyond the projections 24 to effect intradermal or subdermal application of an active substance.

The cap member 14, also a one-piece molded part, comprises an outer wall 28, a parallel inner wall 30 defining a central opening 32 and a radially-directed wall 34. To enable the cap member 14 to be releasably mounted to the inoculator member 12, the outer wall 28 is provided with an integral protrusion 36 on its inner face, which engages the outer wall 20 of the head 18.

The inner end 40 of the wall 32 is chamfered to engage a sloping portion 42 of the wall 22 of the head 18 so as to seal off the opening 32 into which the tines 26 protrude, as seen in Figure 2. The cap 14 is assembled with the handle 12. With the assembly in a vertical orientation, an active liquid material 43 is placed in the opening 32 and a foil covering 44 is adhered over the open end of the opening 32 to hermetically seal the active substance in the cavity into which project the needles 26.

The cap member 14 also includes a ring 46 of absorbent material which is seated against the inner surface of the wall 34 and which has a marking fluid absorbed therein, such as a dye or ink which, when dry, resists removal by washing. When the cap member 14 is assembled to the handle member 12, the projections 24 engage the absorbent pad 46, as seen in Figure 2.

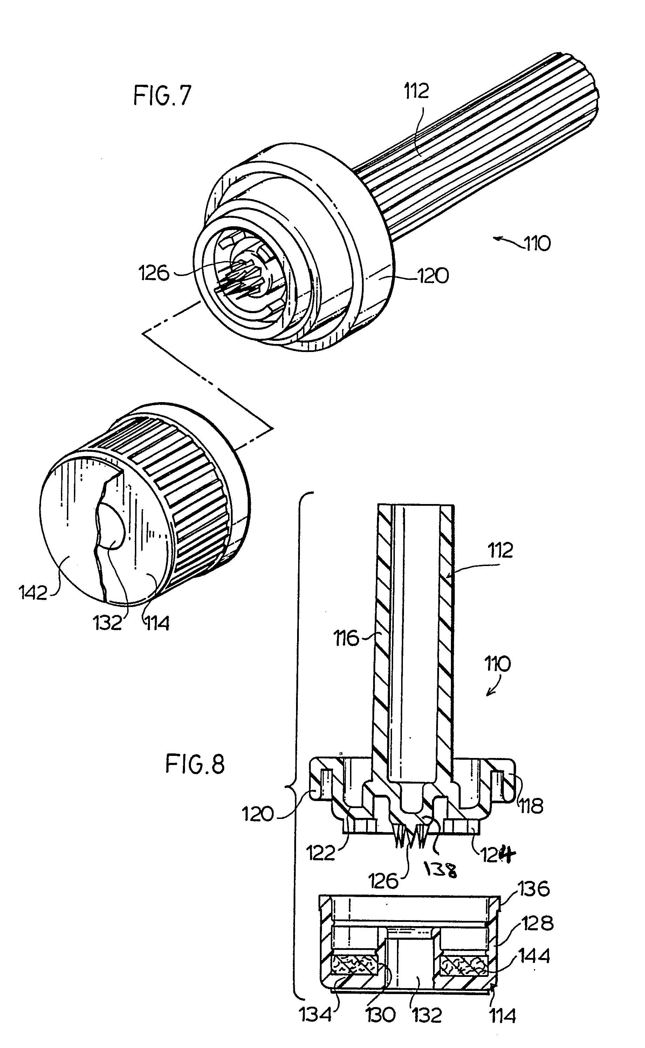

When it is desired to make an application of the active material 43 to a patient's skin, the inoculator member 12 is removed from the cap member 14 and the head 18 is brought into contact with the skin 48 of the patient. The needles 26, which are wetted with the active material 43, penetrate the skin 48 and make an intradermal or subdermal application of the active material, depending on the length of the needles 26. At the same time, the projections 24, which are wetted with marking fluid from contact with the ring 46, engage the outer surface of the skin, as seen in Figure 5, and form a pattern on the skin corresponding to the pattern of the projection 24, as seen in Figure 6.

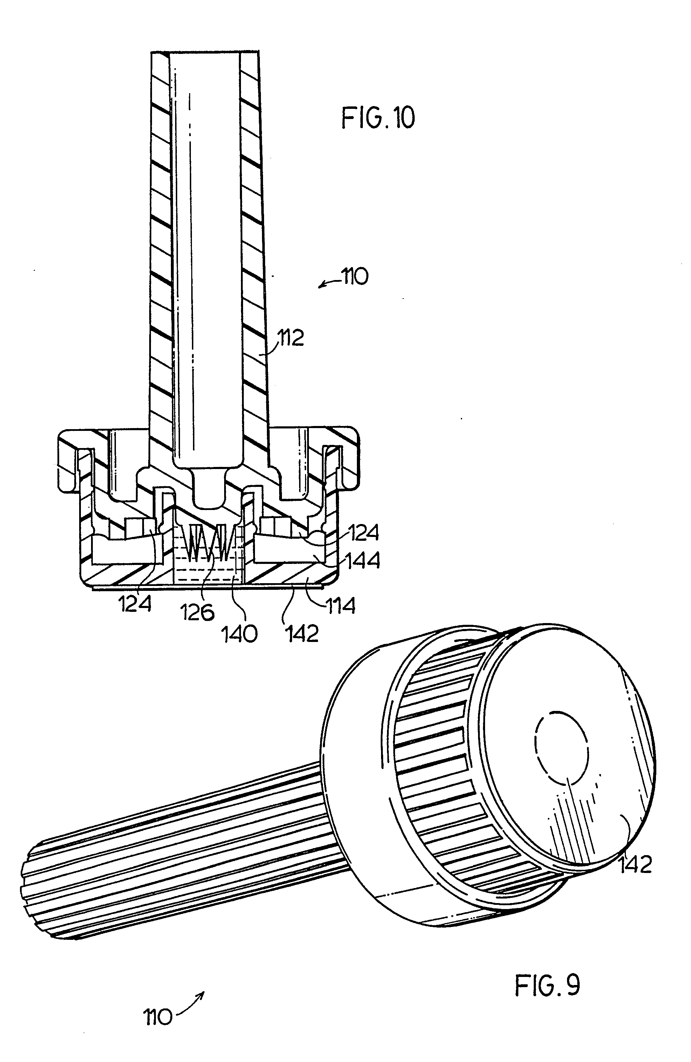

Referring now to Figures 7 to 10 of the drawings, a scarifying device or clinical applicator 110 comprises an inoculator member 112 and a cap member 114. The inoculator member 112, which is a one-piece molded part, comprises an elongate handle 116 in the form of a hollow tube and a head 118 having a cylindrical outer channel structure 120 joined to the handle 116 by a radially-directed flange or wall 122.

A series of projections 124 extend from the wall 122 in the shape and form of an identifying location indicating marking. A plurality of parallel needles or tines 126 protrude from the handle 116 beyond the projections 124 to effect intradermal or subdermal application of an active substance.

The cap member 114, also a one-piece molded part, comprises an outer wall 128, a parallel inner wall 130 defining a central opening 132 and a radially-directed wall 134. To enable the cap member 114 to be releasably mounted to the inoculator member 112, the outer wall 128 is provided with an integral protrusion 136 at its upper extremity, which snap-locks into a corresponding recess formed in the channel member 120 when the outer wall 128 is received in the channel.

At the same time, the inner wall 130 bears against a central cylindrical protrusion 138 on which the tines 126 are mounted. In this way, the opening 132 into which the tines 126 protrude is sealed off.

The cap 114 is assembled with the handle 112. With the assembly in a vertical orientation, an active liquid material 140 is placed in the opening 132 and a foil covering 142 is adhered over the open end of the opening 132 to hermetically seal the active substance in the cavity into which project the needles 126.

The cap member 114 also includes a ring 144 of absorbent material which is seated against the inner surface of the wall 134 and which has a marking fluid absorbed therein, such as a dye or ink which, when dry, resists removal by washing. When the cap member 114 is assembled to the handle member 112 , the projections 124 engage the absorbent pad 146, as seen in Figure 10.

As in the case of the embodiment of Figures 1 to 6, when it is desired to make an application of the active material 140 to a patient's skin, the inoculator member 112 is removed from the cap member and the head 118 is brought into contact with the skin of the patient. The needles 126, which are wetted with the active material 140, penetrate the skin and make an intradermal or subdermal application of the active material, depending on the length of the needles 126. At the same time, the projections 124, which are wetted with marking fluid from contact with the ring 144, engage the outer surface of the skin and form a pattern on the skin corresponding to the pattern of the projections 124.

The pattern of marking fluid, a "happy face" in the illustrated embodiments thereby provides a visual indication of the location of the intradermal or subdermal application but does not, in any way, interfere with the application site.

In summary of this disclosure, the present invention provides a novel scarifying device which enables the location of intradermal or subdermal application of active material to be accurately determined.

## Claims

1. A clinical applicator having a plurality of needles (26, 126) for intradermal or subdermal administration of an active substance (43, 140) characterized in that visual image application means (24, 124) is provided for applying a visual image adjacent the location of and simultaneously with the intradermal or subdermal administration.

2. An applicator as claimed in Claim 1, wherein the visual image application means comprise at least one protrusion (24, 124) from said applicator (10, 110) which engages the skin during said administration.

3. An applicator as claimed in Claim 2, wherein means (46, 144) is provided for applying an image-forming liquid to said at least one protrusion (24, 124).

4. A clinical applicator as claimed in any one of Claims 1 to 3, comprising an inoculator member (12, 112) having an elongate handle (16, 116), from one end of which the plurality of needles (26, 126) extend axially in parallel manner for penetration of skin for administration of the active substance (43, 140) on said needles to an intradermal or subdermal location, and having at least one protrusion (24, 124) extending in the same direction as said plurality of needles (26, 126) and for a distance less than that of said needles; and a cap member (14, 114) comprising an outer ring member (28, 128) having means (36, 136) to releasably assemble the cap member (14, 114) with the inoculator member (12, 112), a source of visual indicator material (46, 144) positioned to be engaged by said at least one protrusion (24, 124) to impart said visual indicator material thereto when said cap member (14, 114) is assembled with said inoculator member (12, 112), and an axially-inwardly directed recess (32, 132) closed at one end and arranged to house said active substance (43, 140) and to receive said needles (26, 126) therein when said cap member (14, 114) is assembled with said inoculator member (12, 112).

5. An applicator as claimed in Claim 4, wherein said protrusion (24, 124) extends from a radially extending annular portion (22, 122) of a generally U-shaped channel member (18, 118) integrally formed at said one end of the handle.

6. An applicator as claimed in Claim 5, wherein the means for releasable assembly comprises an integral ring (36) extending inwardly from the ring member (28) to bear against the outer surface (20) of the U-shaped member (18).

7. An applicator as claimed in Claim 4 or Claim 5 wherein the means for releasable assembly comprises a downwardly opening channel (120) defining a recess for receiving an upper end (136) of the outer ring member (128).

8. An applicator as claimed in any one of Claims 2 to 7, wherein the at least one protrusion (24, 124) comprises an annular protrusion which provides a visual image in the form of a ring surrounding the site of administration.

9. An applicator as claimed in Claim 8, wherein the at least one protrusion (24, 124) comprises a first annular protrusion and arcuate protrusions located between said first annular protrusion and said needles (26, 126) arranged arcuately spaced and coprising two protrusions of short arcuate length and a third protrusion of longer length whereby the visual image has the appearance of a happy face.

10. An applicator as claimed in any one of Claims 3 to 9, comprising an adsorbent material positioned to be engaged by said at least one protrusion and containing a liquid coherent which, when dried on the skin in said visual image, resists removal by washing.

FIG. 2

FIG.1

FIG.5

FIG.6

FIG.3

24    24

24

26

24

4

14

4

FIG. 4

16    12

20    18

24    24

22  42  26  24

36    40

14

28

34    32    30    46

FIG.7

FIG.8

FIG.10

110

112

124

144

124    126    114

140    142

142

110

FIG.9

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | DE-A-3 737 570 (IBERO) <br> * Column 2, lines 6-13; figure * | 1-10 | A 61 B 17/20 <br> A 61 D 1/02 |
| Y | DE-A-2 461 273 (BAUMGARTNER) <br> * Claim 8 * | 1-10 | |
| A | DE-A-2 444 379 (BAUMGARTNER) <br> * Claim 1; page 6, lines 26-28; figures * | 1 | |
| A,D | GB-A-2 068 236 (GALY) <br> * Abstract * | 1 | |
| A,D | US-A-1 699 012 (MAYLOR) <br> * Claim 1; figures * | 1 | |

TECHNICAL FIELDS SEARCHED (Int. Cl.5)

A 61 B
A 61 D

The present search report has been drawn up for all claims

| Place of search | Date of completion of the search | Examiner |
|---|---|---|
| THE HAGUE | 03-05-1990 | GLAS J. |